(19) Europäisches Patentamt
European Patent Office
Office européen des brevets

(11) **EP 4 480 462 A1**

(12) **EUROPEAN PATENT APPLICATION**
published in accordance with Art. 153(4) EPC

(43) Date of publication:
**25.12.2024 Bulletin 2024/52**

(21) Application number: **23779690.9**

(22) Date of filing: **16.03.2023**

(51) International Patent Classification (IPC):
**A61F 13/53** (2006.01)      **A61F 13/531** (2006.01)

(52) Cooperative Patent Classification (CPC):
**A61F 13/53; A61F 13/531**

(86) International application number:
**PCT/JP2023/010409**

(87) International publication number:
**WO 2023/189672 (05.10.2023 Gazette 2023/40)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **29.03.2022 JP 2022053632**

(71) Applicant: **SUMITOMO SEIKA Chemicals Co., Ltd.
Kako-gun, Hyogo 675-0145 (JP)**

(72) Inventor: **OKAZAWA Shiho
Himeji-shi, Hyogo 672-8076 (JP)**

(74) Representative: **Vossius & Partner
Patentanwälte Rechtsanwälte mbB
Siebertstrasse 3
81675 München (DE)**

(54) **WATER ABSORBENT RESIN PARTICLES AND ABSORBENT ARTICLE**

(57)    The present invention relates to a water-absorbent resin particle containing a crosslinked polymer having, as a monomer unit, at least one ethylenically unsaturated monomer selected from the group consisting of (meth)acrylic acid and a salt thereof, in which a ratio of the (meth)acrylic acid and the salt thereof is 70% to 100% by mol with respect to a total amount of the monomer unit in the crosslinked polymer, a water retention capacity of the water-absorbent resin particle in a physiological saline is 40 g/g or more, a sum of a water retention capacity (g/g) of the water-absorbent resin particle in the physiological saline and a water absorption amount (g/g) of the water-absorbent resin particle under a load of 6.21 kPa is 56 or more, and a water absorption rate of the water-absorbent resin particle is 50 seconds or less.

**Fig.1**

## Description

### Technical Field

The present invention relates to a water-absorbent resin particle and an absorbent article.

### Background Art

The water-absorbent resin particles are used for various applications, and are particularly used for sanitary products such as diapers. As such water-absorbent resin particles, Patent Literature 1 discloses a superabsorbent polymer that contains an encapsulated blowing agent and has a vortex time of 40 seconds or less.

Citation List

### Patent Literature

[Patent Literature 1] Japanese Unexamined Patent Publication No. 2007-514833

### Summary of Invention

### Technical Problem

In an absorber used in an absorbent article such as a diaper, pulp is used in addition to water-absorbent resin particles. For the purpose of forest conservation, it is required to reduce the use amount of pulp, and in accordance with this, it is required to reduce a content of pulp in the absorber or to eliminate pulp from the absorber. In the absorber containing the water-absorbent resin particles at a high proportion (for example, a proportion of 90% by mass or more), swollen gels are likely to move when a load is applied after urination, and the swollen gels may leak.
An object of the present invention is to provide a water-absorbent resin particle capable of reducing gel leakage even though an absorber contains the water-absorbent resin particle at a high proportion.

### Solution to Problem

One aspect of the present invention relates to a water-absorbent resin particle containing a crosslinked polymer having, as a monomer unit, at least one ethylenically unsaturated monomer selected from the group consisting of (meth) acrylic acid and a salt thereof, in which a ratio of the (meth)acrylic acid and the salt thereof is 70% to 100% by mol with respect to a total amount of the monomer unit in the crosslinked polymer, a water retention capacity of the water-absorbent resin particle in a physiological saline is 40 g/g or more, a sum of a water retention capacity (g/g) of the water-absorbent resin particle in the physiological saline and a water absorption amount (g/g) of the water-absorbent resin particle under a load of 6.21 kPa is 56 g/g or more, and a water absorption rate of the water-absorbent resin particle is 50 seconds or less.
Another aspect of the present invention relates to an absorber including the above-mentioned water-absorbent resin particle. Another aspect of the present invention relates to an absorbent article containing the above-mentioned absorber.

### Advantageous Effects of Invention

According to the present invention, it is possible to provide the water-absorbent resin particle capable of reducing gel leakage even though an absorber contains the water-absorbent resin particle at a high proportion.

### Brief Description of Drawings

Fig. 1 is a schematic cross-sectional view showing an embodiment of a water-absorbent sheet.
Fig. 2 is a schematic cross-sectional view showing an embodiment of an absorbent article.
Fig. 3 is a schematic diagram showing a measurement device for the water absorption amount of water-absorbent resin particles under a load.

**Description of Embodiments**

Hereinafter, some embodiments of the present invention will be described in detail. However, the present invention is not limited to the following embodiments.

In the present specification, "acrylic" and "methacrylic" are collectively referred to as "(meth)acrylic". "Acrylate" and "methacrylate" are also referred to as "(meth)acrylate". In a numerical value range described in a stepwise manner in the present specification, an upper limit value or a lower limit value of a numerical value range in a certain step can be optionally combined with an upper limit value or a lower limit value of a numerical value range in another step. In a numerical value range described in the present specification, the upper limit value or the lower limit value of the numerical value range may be replaced with the value shown in the examples. For materials exemplified in the present specification, one kind may be used alone, or two or more kinds may be used in combination. In a case where there are a plurality of substances corresponding to each of components in a composition, a content of each of the components in the composition means the total amount of the plurality of substances present in the composition unless otherwise specified. In the present specification, "physiological saline" is a sodium chloride aqueous solution having a concentration of 0.9% by mass, and the concentration of 0.9% by mass is based on the mass of the physiological saline.

A water-absorbent resin particle according to the present embodiment includes a crosslinked polymer having, as a monomer unit, at least one ethylenically unsaturated monomer selected from the group consisting of (meth)acrylic acid and a salt thereof, in which a ratio of the (meth)acrylic acid and the salt thereof is 70% to 100% by mol with respect to a total amount of a monomer unit in the crosslinked polymer. A water retention capacity of the water-absorbent resin particle in a physiological saline is 40 g/g or more, a sum of a water retention capacity (g/g) of the water-absorbent resin particle in the physiological saline and a water absorption amount (g/g) of the water-absorbent resin particle under a load of 6.21 kPa is 56 g/g or more, and the water absorption rate of the water-absorbent resin particle is 50 seconds or less.

The water retention capacity of the water-absorbent resin particle in the physiological saline is 40 g/g or more. The water retention capacity may be 42 g/g or more, 44 g/g or more, 46 g/g or more, or 48 g/g or more. The upper limit of the water retention capacity may be, for example, 70 g/g or less, 65 g/g or less, 60 g/g or less, 55 g/g or less, or 50 g/g or less. The water retention capacity may be, for example, 40 g/g or more and 70 g/g or less, 40 g/g or more and 65 g/g or less, 40 g/g or more and 60 g/g or less, 40 g/g or more and 55 g/g or less, 40 g/g or more and 50 g/g or less, 42 g/g or more and 70 g/g or less, 42 g/g or more and 65 g/g or less, 42 g/g or more and 60 g/g or less, 44 g/g or more and 55 g/g or less, or 44 g/g or more and 50 g/g or less. Provided that the water retention capacity is within the above-mentioned range, the better effect of reducing gel leakage in a case of using an absorber that contains the water-absorbent resin particle at a high proportion is achieved. The water retention capacity can be measured by a method described in Examples described later.

The water absorption amount under a load of 6.21 kPa (hereinafter, also referred to as the "water absorption amount under the load 6.21 kPa") is a value measured by a water absorption test carried out as the following procedure: the water-absorbent resin particles are arranged on a liquid-permeable sheet (mesh sheet), which is placed on a measurement table having a through-hole; and the water-absorbent resin particles are allowed to absorb a physiological saline supplied from the through-hole without pressure while a load of 6.21 kPa is applied to the water-absorbent resin particles. Usually, an inner diameter of the through-hole is 2 mm. The amount of the water-absorbent resin particles used in the test is 0.100 g, and the water-absorbent resin particles in this amount are uniformly arranged in a cylinder at a position directly above the through-hole, which has an inner diameter of 20 mm serving as the center. The weight [g/g] (the weight per 1 g of the water-absorbent resin particle) of the physiological saline absorbed by the water-absorbent resin particle within 60 minutes from the start of the water absorption is defined as the water absorption amount under the load 6.21 kPa. Details of the test conditions will be described in Examples later.

The sum of the water retention capacity (g/g) of the physiological saline and the water absorption amount under the load 6.21 kPa (hereinafter, also referred to as "the water retention capacity + the water absorption amount under load 6.21 kPa") is 56 g/g or more. The water retention capacity + the water absorption amount under load 6.21 kPa may be 57 g/g or more, 58 g/g or more, 59 g/g or more, or 60 g/g or more. The upper limit of the water retention capacity + the water absorption amount under load 6.21 kPa may be, for example, 80 g/g or less, 75 g/g or less, 70 g/g or less, or 65 g/g or less. The water retention capacity + the water absorption amount under load 6.21 kPa may be, for example, 56 g/g or more and 80 g/g or less, 56 g/g or more and 75 g/g or less, 56 g/g or more and 70 g/g or less, 56 g/g or more and 65 g/g or less, 57 g/g or more and 80 g/g or less, 57 g/g or more and 75 g/g or less, 57 g/g or more and 70 g/g or less, 57 g/g or more and 65 g/g or less, 58 g/g or more and 80 g/g or less, 58 g/g or more and 75 g/g or less, 58 g/g or more and 70 g/g or less, 58 g/g or more and 65 g/g or less, 59 g/g or more and 80 g/g or less, 59 g/g or more and 75 g/g or less, 59 g/g or more and 70 g/g or less, 59 g/g or more and 65 g/g or less, 60 g/g or more and 80 g/g or less, 60 g/g or more and 75 g/g or less, 60 g/g or more and 70 g/g or less, or 60 g/g or more and 65 g/g or less. Provided that the water retention capacity + the water absorption amount under load 6.21 kPa is within the above-mentioned range, the better effect of reducing gel leakage in a case of using an absorber that contains the water-absorbent resin particle at a high proportion is achieved.

The water absorption amount under the load 6.21 kPa may be 5 g/g or more, 7 g/g or more, 10 g/g or more, or 13 g/g or more. The water absorption amount under the load 6.21 kPa may be, for example, 30 g/g or less, 25 g/g or less, 20 g/g or less, or 15 g/g or less. The water absorption amount under the load 6.21 kPa may be 5 g/g or more and 30 g/g or less, 7 g/g or more and 25 g/g or less, 10 g/g or more and 20 g/g or less, or 13 g/g or more and 15 g/g or less. Provided that the water absorption amount under the load 6.21 kPa is within the above-mentioned range, the better effect of reducing gel leakage in a case of using an absorber that contains the water-absorbent resin particle at a high proportion is achieved.

The water absorption rate of the water-absorbent resin particle is 50 seconds or less. The water absorption rate may be 48 seconds or less, 46 seconds or less, 44 seconds or less, 42 seconds or less, or 40 seconds or less. The water absorption rate may be, for example, 25 seconds or more, 30 seconds or more, or 35 seconds or more. Provided that the water absorption rate is within the above-mentioned range, the better effect of reducing gel leakage in a case of using an absorber that contains the water-absorbent resin particle at a high proportion is achieved.

The water absorption rate is according to a Vortex method, and can be measured by the following method. $50 \pm 0.1$ g of a physiological saline, the temperature of which is adjusted to a temperature of $25 \pm 0.2°C$ in a constant temperature water tank, is measured in a beaker having a volume of 100 mL. Subsequently, a vortex is generated by stirring at a rotation speed of 600 rpm using a magnetic stirrer bar (8 mm$\varphi \times$ 30 mm, without ring). $2.0 \pm 0.002$ g of the water-absorbent resin particles is added to the physiological saline at one time. The time period [seconds] from the time when the water-absorbent resin particles are added to the time when the vortex on the liquid surface is converged is measured, and the time period is recorded as the water absorption rate of the water-absorbent resin particle.

The water absorption amount of the water-absorbent resin particle under a load of 2.07 kPa (hereinafter, also referred to as the "water absorption amount under load 2.07 kPa") may be, for example, 15 g/g or more, 20 g/g or more, 25 g/g or more, 30 g/g or more, or 35 g/g or more. The upper limit of the water absorption amount under load 2.07 kPa may be, for example, 50 g/g or less, 45 g/g or less, or 40 g/g or less.

The water absorption amount under load 2.07 kPa is measured in the same manner as the water absorption amount under the load 6.21 kPa, except that the load during the water absorption test is 2.07 kPa. Details of the test conditions will be described in Examples later.

The sum of the water retention capacity (g/g) of the physiological saline and the water absorption amount under load 2.07 kPa (g/g) (hereinafter, also referred to as "the water retention capacity + the water absorption amount under load 2.07 kPa") may be, for example, 65 g/g or more, 70 g/g or more, 75 g/g or more, or 80 g/g or more. The water retention capacity + the water absorption amount under load 2.07 kPa may be 100 g/g or less, 95 g/g or less, 90 g/g or less, or 85 g/g or less.

The median particle diameter of the water-absorbent resin particle may be, for example, 200 $\mu$m or more, 250 $\mu$m or more, or 300 $\mu$m or more. The median particle diameter of the water-absorbent resin particle may be, for example, 600 $\mu$m or less, 500 $\mu$m or less, 400 $\mu$m or less, or 350 $\mu$m or less. The median particle diameter may be, for example, 200 $\mu$m or more and 600 $\mu$m or less, 200 $\mu$m or more and 500 $\mu$m or less, 200 $\mu$m or more and 400 $\mu$m or less, 200 $\mu$m or more and 350 $\mu$m or less, 250 $\mu$m or more and 600 $\mu$m or less, 250 $\mu$m or more and 500 $\mu$m or less, 250 $\mu$m or more and 400 $\mu$m or less, 250 $\mu$m or more and 350 $\mu$m or less, 300 $\mu$m or more and 600 $\mu$m or less, 300 $\mu$m or more and 500 $\mu$m or less, 300 $\mu$m or more and 400 $\mu$m or less, or 300 $\mu$m or more and 350 $\mu$m or less.

The median particle diameter can be measured by the following method. JIS standard sieves are combined in the following order from the upper side: sieves having openings of 850 $\mu$m, 600 $\mu$m, 500 $\mu$m, 425 $\mu$m, 300 $\mu$m, 250 $\mu$m, and 180 $\mu$m, and a tray. 5 g of the water-absorbent resin particles are placed onto the combined uppermost sieve and classified by using a continuous fully automatic sonic vibration-type sieving measuring apparatus (Robot shifter RPS-205, manufactured by Seishin Enterprise Co., Ltd.). After classification, the mass of the particles remaining on each sieve is calculated as a mass percentage with respect to the total amount to determine a particle size distribution. The relationship between the opening of the sieve and the integrated value of the mass percentage of the particles remaining on the sieve is plotted on logarithmic probability paper by integrating in the order from the one having the largest particle diameter on the sieve with respect to this particle size distribution. By connecting the plots on the probability paper with a straight line, the particle diameter corresponding to the cumulative mass percentage of 50% by mass is obtained as the median particle diameter. Other details of the test conditions will be described in Examples described later.

A shape of the water-absorbent resin particle is not particularly limited, and may be, for example, a substantially spherical shape, a crushed shape, or a granular shape or may be a particle formed such that primary particles with each of these shapes aggregate.

The water-absorbent resin particle according to the present embodiment contains a crosslinked polymer having, as a monomer unit, at least one ethylenically unsaturated monomer selected from the group consisting of (meth)acrylic acid and a salt thereof.

The crosslinked polymer may have another ethylenically unsaturated monomer in addition to (meth)acrylic acid and a salt thereof, as a monomer unit. The other ethylenically unsaturated monomer may be contained in at least one compound selected from the group consisting of 2-(meth)acrylamide-2-methylpropanesulfonic acid and a salt thereof, (meth)acrylamide, N,N-dimethyl (meth)acrylamide, 2-hydroxyethyl (meth)acrylate, N-methylol (meth)acrylamide, polyethylene glycol mono(meth)acrylate, N,N-diethylaminoethyl (meth)acrylate, N,N-diethylaminopropyl (meth)acrylate, and diethylaminopropyl (meth)acrylamide. In a case where the other ethylenically unsaturated monomer has an amino group, the amino group may be quaternized.

In a case where the monomer unit in the crosslinked polymer has an acid group, the monomer unit may be used in a polymerization reaction after neutralizing the acid group with an alkaline neutralizing agent. The neutralization degree of the ethylenically unsaturated monomer by the alkaline neutralizing agent may be 50% to 100% by mol, 60% to 90% by mol, 70% to 85% by mol, or 75% to 80% by mol of the acid group in the ethylenically unsaturated monomer, from the viewpoint of increasing an osmotic pressure of the obtained water-absorbent resin particle, and further enhancing water-absorbent characteristics. Examples of the alkaline neutralizing agent include alkali metal salts such as sodium hydroxide, sodium carbonate, sodium hydrogen carbonate, potassium hydroxide, and potassium carbonate; and ammonia. The alkaline neutralizing agent may be used alone, or two or more kinds thereof may be used in combination. The alkaline neutralizing agent may be used in the form of an aqueous solution to simplify the neutralization operation.

In the crosslinked polymer, the ratio of (meth)acrylic acid and a salt thereof is 70% to 100% by mol with respect to the total amount of the monomer units in the crosslinked polymer, and may be, for example, 80% to 100% by mol or 90% to 100% by mol.

The water-absorbent resin particle can be produced by, for example, a method including a step of polymerizing a monomer including the above-mentioned ethylenically unsaturated monomer in a reaction solution containing the monomer and a radical polymerization initiator. As the radical polymerization initiator, a water-soluble radical polymerization initiator can be used.

Examples of methods of polymerization include a reverse phase suspension polymerization method, an aqueous solution polymerization method, a bulk polymerization method, and a precipitation polymerization method. The polymerization method may be the reverse phase suspension polymerization method or the aqueous solution polymerization method from the viewpoints of facilitating securement of good water-absorbent characteristics of the obtained water-absorbent resin particle and control of a polymerization reaction. In the following, as a method for polymerizing the ethylenically unsaturated monomer, a reverse phase suspension polymerization method will be described as an example.

The ethylenically unsaturated monomer is usually preferably used as an aqueous solution. The concentration of the ethylenically unsaturated monomers in the aqueous solution containing the ethylenically unsaturated monomers (hereinafter, simply referred to as a "monomer aqueous solution") may be 20% by mass or more and a saturated concentration or less, 25% to 70% by mass, or 30% to 55% by mass. Examples of the water used in the aqueous solution include tap water, distilled water, and ion-exchanged water.

Examples of the radical polymerization initiator include persulfates such as potassium persulfate, ammonium persulfate, and sodium persulfate; azo compounds such as 2,2'-azobis(2-amidinopropane) dihydrochloride, 2,2'-azobis [2-(N-phenylamidino)propane] dihydrochloride, 2,2'-azobis[2-(N-allylamidino)propane] dihydrochloride, 2,2'-azobis [2-(2-imidazolin-2-yl)propane] dihydrochloride, 2,2'-azobis{2-[1-(2-hydroxyethyl)-2-imidazolin-2-yl]propane} dihydrochloride, 2,2'-azobis{2-methyl-N-[1,1-bis(hydroxymethyl)-2-hydroxyethyl]propionamide}, 2,2'-azobis[2-methyl-N-(2-hydroxyethyl)-propionamide], and 4,4'-azobis(4-cyanovaleric acid); peroxides such as methyl ethyl ketone peroxide, methyl isobutyl ketone peroxide, di-t-butyl peroxide, t-butyl cumyl peroxide, t-butyl peroxyacetate, t-butyl peroxyisobutyrate, t-butyl peroxypivalate, and hydrogen peroxide; and other water-soluble radical polymerization initiators.

The radical polymerization initiator may be used alone, or two or more kinds thereof may be used in combination. The radical polymerization initiator is preferably at least one selected from the group consisting of a persulfate and a peroxide and more preferably a persulfate, from the viewpoint that the water-absorbent resin particle having both the water retention capacity and the water retention capacity + the water absorption amount under load 6.21 kPa within the above-mentioned range is more easily obtained.

The use amount of the radical polymerization initiator may be 0.28 mmol or more with respect to 1 mol of the ethylenically unsaturated monomer, from the viewpoint that the water-absorbent resin particle having both the water retention capacity and the water retention capacity + the water absorption amount under load 6.21 kPa within the above-mentioned range is more easily obtained. The use amount of the radical polymerization initiator may be, for example, 10.00 mmol or less, 5.00 mmol or less, 1.00 mmol or less, 0.80 mmol or less, or 0.70 mmol or less with respect to 1 mol of the ethylenically unsaturated monomer, from the viewpoint of easily reducing the occurrence of a rapid polymerization reaction. The use amount of the radical polymerization initiator may be, for example, 0.28 mmol or more and 10.00 mmol or less.

Provided that the radical polymerization initiator is at least one selected from the group consisting of a persulfate and a peroxide, or the use amount of the radical polymerization initiator is 0.28 mmol or more with respect to 1 mol of the ethylenically unsaturated monomer, the radical polymerization initiator is likely to remain as unreacted in a hydrogel-like polymer obtained by the polymerization reaction. Entanglement of molecular chains is increased in the vicinity of the surface because the hydrogel-like polymer is heated with the unreacted radical polymerization initiator remaining, and water-absorbent resin particles having a large amount of physical crosslinking in the vicinity of the surface thus tends to be obtained. Adjusting the degree of crosslinking by the physical crosslinking in the vicinity of the surface enables an increase in the water absorption amount under the load 6.21 kPa even while giving the water-absorbent resin particle a high water retention capacity. As a result, the water-absorbent resin particle having both the water retention capacity and the water retention capacity + the water absorption amount under the load 6.21 kPa within the above-mentioned range are more easily obtained.

In the reverse phase suspension polymerization method, a monomer aqueous solution is dispersed in a hydrocarbon dispersion medium in the presence of a surfactant, and polymerization of the ethylenically unsaturated monomer can be performed using a radical polymerization initiator or the like.

Examples of the surfactant include a nonionic surfactant, an anionic surfactant, and other surfactants. Examples of the nonionic surfactant include sorbitan fatty acid esters, polyglycerin fatty acid esters, sucrose fatty acid esters, polyoxyethylene sorbitan fatty acid esters, polyoxyethylene glycerin fatty acid esters, sorbitol fatty acid esters, polyoxyethylene sorbitol fatty acid esters, polyoxyethylene alkyl ethers, polyoxyethylene alkylphenyl ethers, polyoxyethylene castor oil, polyoxyethylene hydrogenated castor oil, alkylallyl formaldehyde condensed polyoxyethylene ethers, polyoxyethylene polyoxypropylene block copolymers, polyoxyethylene polyoxypropyl alkyl ethers, and polyethylene glycol fatty acid esters. Examples of the anionic surfactant include fatty acid salts, alkylbenzene sulfonate, alkylmethyl taurate, polyoxyethylene alkylphenyl ether sulfuric acid ester salts, polyoxyethylene alkyl ether sulfonic acid salts, phosphoric acid esters of polyoxyethylene alkyl ethers, and phosphoric acid esters of polyoxyethylene alkyl allyl ethers. The surfactant may be used alone, or two or more kinds thereof may be used in combination.

From the viewpoint that the state of the W/O type reverse phase suspension is good, and the water-absorbent resin particle having a suitable particle diameter is easily obtained, and the viewpoint of industrial availability, the surfactant may contain at least one compound selected from the group consisting of sorbitan fatty acid esters, polyglycerin fatty acid esters, and sucrose fatty acid esters. The surfactant may include sucrose fatty acid esters or may include sucrose stearic acid esters from the viewpoint that water-absorbent characteristics of the obtained water-absorbent resin particle are then easily improved.

The use amount of the surfactant may be 0.05 to 10 parts by mass, 0.08 to 5 parts by mass, or 0.1 to 3 parts by mass, with respect to 100 parts by mass of the monomer aqueous solution, from the viewpoint of obtaining sufficient effect for the use amount and economic efficiency.

For the reverse phase suspension polymerization, a polymeric dispersant may be used in combination with the above-mentioned surfactant. Examples of the polymeric dispersant include maleic acid anhydride-modified polyethylene, maleic acid anhydride-modified polypropylene, a maleic acid anhydride-modified ethylene-propylene copolymer, a maleic acid anhydride-modified EPDM (ethylene propylene diene terpolymer), maleic acid anhydride-modified polybutadiene, a maleic acid anhydride-ethylene copolymer, a maleic acid anhydride-propylene copolymer, a maleic acid anhydride-ethylene-propylene copolymer, a maleic acid anhydride-butadiene copolymer, polyethylene, polypropylene, an ethylene-propylene copolymer, oxidized polyethylene, oxidized polypropylene, an oxidized ethylene-propylene copolymer, an ethylene-acrylic acid copolymer, ethyl cellulose, and ethyl hydroxyethyl cellulose. The polymeric dispersant may be used alone or may be used in combination of two or more kinds thereof. From the viewpoint of better dispersion stability of monomers, the polymeric dispersant may include at least one selected from the group consisting of maleic acid anhydride-modified polyethylene, maleic acid anhydride-modified polypropylene, a maleic acid anhydride-modified ethylene-propylene copolymer, a maleic acid anhydride-ethylene copolymer, a maleic acid anhydride-propylene copolymer, a maleic acid anhydride-ethylene-propylene copolymer, polyethylene, polypropylene, an ethylene-propylene copolymer, oxidized polyethylene, oxidized polypropylene, and an oxidized ethylene-propylene copolymer.

The use amount of the polymeric dispersant may be 0.05 to 10 parts by mass, 0.08 to 5 parts by mass, or 0.1 to 3 parts by mass, with respect to 100 parts by mass of the monomer aqueous solution, from the viewpoint of obtaining sufficient effect for the use amount and economic efficiency.

The hydrocarbon dispersion medium may contain at least one compound selected from the group consisting of chain aliphatic hydrocarbons having 6 to 8 carbon atoms and alicyclic hydrocarbons having 6 to 8 carbon atoms. Examples of the hydrocarbon dispersion medium include chain aliphatic hydrocarbons such as n-hexane, n-heptane, 2-methylhexane, 3-methylhexane, 2,3-dimethylpentane, 3-ethylpentane, and n-octane; alicyclic hydrocarbons such as cyclohexane, methylcyclohexane, cyclopentane, methylcyclopentane, trans-1,2-dimethylcyclopentane, cis-1,3-dimethylcyclopentane, and trans-1,3-dimethylcyclopentane; and aromatic hydrocarbons such as benzene, toluene, and xylene. The hydrocarbon dispersion medium may be used alone, or two or more kinds thereof may be used in combination.

From the viewpoint of industrial availability and stable quality, the hydrocarbon dispersion medium may contain at least one selected from the group consisting of n-heptane and cyclohexane. From the same viewpoint, as the mixture of the above-mentioned hydrocarbon dispersion medium, for example, a commercially available Exxsol Heptane (manufactured by ExxonMobil: containing 75% to 85% of n-heptane and isomeric hydrocarbons) may be used.

The use amount of the hydrocarbon dispersion medium may be 30 to 1,000 parts by mass, 40 to 500 parts by mass, or 50 to 300 parts by mass, with respect to 100 parts by mass of the monomer aqueous solution, from the viewpoint that polymerization heat is appropriately removed, and a polymerization temperature is easily controlled. Provided that the use amount of the hydrocarbon dispersion medium is 30 parts by mass or more, the polymerization temperature tends to be easily controlled. Provided that the use amount of the hydrocarbon dispersion medium is 1000 parts by mass or less, the productivity of polymerization tends to be improved, which is economical.

Crosslinking by self-crosslinking may occur during polymerization, but the crosslinking may be carried out by further using an internal crosslinking agent. In a case where an internal crosslinking agent is used, water-absorbent characteristics of the water-absorbent resin particle are easily controlled. The internal crosslinking agent is usually added to a reaction solution during the polymerization reaction. Examples of the internal crosslinking agent include di- or tri(meth) acrylic acid esters of polyols such as ethylene glycol, propylene glycol, trimethylolpropane, glycerin, polyoxyethylene glycol, polyoxypropylene glycol, and polyglycerin; unsaturated polyesters obtained by reacting the above-mentioned polyols with unsaturated acids (such as maleic acid and fumaric acid); bis(meth)acrylamides such as N,N'-methylene bis(meth)acrylamide; di- or tri(meth)acrylic acid esters obtained by reacting polyepoxide with (meth)acrylic acid; di(meth) acrylic acid carbamyl esters obtained by reacting polyisocyanate (such as tolylene diisocyanate and hexamethylene diisocyanate) with hydroxyethyl (meth)acrylate; compounds having two or more polymerizable unsaturated groups such as allylated starch, allylated cellulose, diallyl phthalate, N,N',N''-triallyl isocyanurate, and divinylbenzene; polyglycidyl compounds such as (poly)ethylene glycol diglycidyl ether, (poly)propylene glycol diglycidyl ether, (poly)glycerin diglycidyl ether, (poly)glycerin triglycidyl ether, (polypropylene glycol polyglycidyl ether, and polyglycerol polyglycidyl ether; haloepoxy compounds such as epichlorohydrin, epibromohydrin, and $\alpha$-methyl epichlorohydrin; compounds having two or more reactive functional groups such as isocyanate compounds (2,4-tolylene diisocyanate and hexamethylene diisocyanate), and other internal crosslinking agents. These internal crosslinking agents may be used alone or two or more kinds thereof may be used in combination. From the viewpoint that the uniformity of the crosslinking in the water-absorbent resin particle is likely to be increased, the internal crosslinking agent may contain a polyglycidyl compound or a diglycidyl ether compound or may contain at least one selected from the group consisting of a (poly)ethylene glycol diglycidyl ether, a (poly)propylene glycol diglycidyl ether, and a (poly)glycerin diglycidyl ether.

The use amount of the internal crosslinking agent may be 0 mmol or more, 0.02 mmol or more, 0.03 mmol or more, 0.04 mmol or more, or 0.05 mmol or more, and may be 0.1 mol or less per 1 mol of the ethylenically unsaturated monomer, from the viewpoints that water-soluble properties are suppressed by the appropriate crosslinking of the obtained polymer, and a sufficient water absorption amount is thus easily obtained.

An aqueous phase containing an ethylenically unsaturated monomer, a radical polymerization initiator, and as necessary, an internal crosslinking agent, and the like; and an oil phase containing a hydrocarbon dispersant, and as necessary, a surfactant and a polymeric dispersant, and the like can be mixed and heated under stirring to carry out the reverse phase suspension polymerization in a water-in-oil system.

A monomer aqueous solution containing the ethylenically unsaturated monomer is dispersed in a hydrocarbon dispersion medium in the presence of a surfactant (as necessary, additionally a polymeric dispersant) when the reverse phase suspension polymerization is performed. At this time, the timing of adding the surfactant, the polymeric dispersant, or the like may be either before or after the addition of the monomer aqueous solution as long as the polymerization reaction is not yet started.

From the viewpoint of easily reducing the amount of the hydrocarbon dispersion medium remaining in the obtained water-absorbent resin particle, polymerization may be performed after the monomer aqueous solution is dispersed in the hydrocarbon dispersion medium in which the polymeric dispersant is dispersed, followed by further dispersing the surfactant.

The reverse phase suspension polymerization can be performed in one stage, or in multiple stages of two or more stages. The reverse phase suspension polymerization may be performed in two or three stages from the viewpoint of increasing productivity.

In a case where the reverse phase suspension polymerization is performed in multiple stages of two or more stages, a first stage of the reverse phase suspension polymerization method is performed, followed by the addition of the ethylenically unsaturated monomer to the reaction mixture, which is obtained in the first polymerization reaction, to be mixed, and second and subsequent stages of the reverse phase suspension polymerization method may be performed in the same method as the first stage. For the reverse phase suspension polymerization at each stage of the second and subsequent stages, in addition to the ethylenically unsaturated monomer, the above-mentioned radical polymerization initiator may be added within the range of a molar ratio of each component with respect to the above-mentioned ethylenically unsaturated monomer, based on the amount of the ethylenically unsaturated monomer added during the second and subsequent stages of the reverse phase suspension polymerization method, to perform the reverse phase suspension polymerization. For the reverse phase suspension polymerization at each stage of the second and subsequent stages, an internal crosslinking agent may be used as necessary. In a case of using the internal crosslinking agent, the internal crosslinking agent may be added within the range of a molar ratio of each component with respect to the above-mentioned ethylenically unsaturated monomer based on the amount of the ethylenically unsaturated monomer provided in each stage, to perform the reverse phase suspension polymerization.

A temperature of the polymerization reaction may vary depending on the radical polymerization initiator to be used. The temperature of the polymerization reaction may be 20°C to 150°C or 40°C to 120°C, from the viewpoints that the polymerization is allowed to promptly proceed to shorten a polymerization time to increase economic efficiency increases, and polymerization heat is easily removed to smoothly perform the reaction. The reaction time may be, for example, 0.5 to 4 hours. The completion of the polymerization reaction can be confirmed upon the stopping of the temperature rise in the reaction system, for example. Thus, the polymer of the ethylenically unsaturated monomer is usually obtained as a hydrogel-like polymer.

The hydrogel-like polymer obtained after the polymerization may be heated with water being present on a surface to some extent. The hydrogel-like polymer may be heated in a state in which the moisture percentage of the hydrogel-like polymer is within a range of 145% by mass to 220% by mass when the heating is started, from the viewpoints that the water-absorbent resin particles having a large amount of physical crosslinking in the vicinity of the surface are easily obtained, and the water-absorbent resin particles having both the water retention capacity and the water retention capacity + the water absorption amount under the load 6.21 kPa within the above-mentioned range are more easily obtained. The moisture percentage of the hydrogel-like polymer at the start of heating may be the moisture percentage of the hydrogel-like polymer at the time when the heating is started at a temperature of 70°C or higher. The moisture percentage of the hydrogel-like polymer may be, for example, 150% by mass to 200% by mass or 155% by mass to 180% by mass.

The moisture percentage (% by mass) of the hydrogel-like polymer is calculated by the following expression.

$$\text{Moisture percentage} = (Ww/Ws) \times 100$$

Ww: Water amount of the hydrogel-like polymer obtained by adding water amount added and water amount used if necessary when mixing a radical polymerization initiator, a flocculant, a surface crosslinking agent, or the like to an amount obtained by subtracting water amount discharged to the outside of the system in the step such as a drying step from water amount contained in a monomer aqueous solution before polymerization in entire polymerization step.

Ws: Solid content calculated from the charged amount of materials such as the ethylenically unsaturated monomer, the crosslinking agent, and the radical polymerization initiator, which constitute the hydrogel-like polymer.

The moisture percentage of the hydrogel-like polymer can be controlled within the above-mentioned range by, for example, a method of adding water to the hydrogel-like polymer obtained after the polymerization reaction, a method of allowing water to remain preliminarily in the hydrogel-like polymer obtained by the polymerization reaction, or a method of combining aforementioned methods.

The heating temperature when the hydrogel-like polymer is heated may be, for example, 70°C or higher and 250°C or lower. The lower limit of the heating temperature may be, for example, 90°C or higher, 110°C or higher, or 120°C or higher. Examples of a method of heating the hydrogel-like polymer include a method of immersing a reaction container containing the hydrogel-like polymer in an oil bath set to the above-mentioned heating temperature.

The hydrogel-like polymer that is obtained after the polymerization may be heated in the presence of the radical polymerization initiator, from the viewpoints that the water-absorbent resin particle having a large amount of physical crosslinking in the vicinity of the surface are easily obtained, and the water-absorbent resin particle having both the water retention capacity and the water retention capacity + the water absorption amount under load 6.21 kPa within the above-mentioned range are more easily obtained. The radical polymerization initiator may be an unreactive product of the radical polymerization initiator used in the polymerization step, or may be a radical polymerization initiator added to the hydrogel-like polymer after the polymerization reaction. In a case where the radical polymerization initiator is added to the hydrogel-like polymer after the polymerization reaction, the radical polymerization initiator may be added together with water.

The hydrogel-like polymer may be heated to remove at least part of water. For example, the water can be removed by heating the hydrogel-like polymer in a state of being dispersed in a hydrocarbon dispersion medium from the outside to perform azeotropic distillation and refluxing the hydrocarbon dispersion medium.

Crosslinking may be obtained on surface layer portions (surface crosslinking) of the hydrogel-like polymer. The water-absorbent characteristics of the water-absorbent resin particle are easily controlled by performing the surface crosslinking.

Examples of crosslinking agents for performing the surface crosslinking (surface crosslinking agents) include compounds having two or more reactive functional groups. Examples of the crosslinking agent include polyols such as ethylene glycol, propylene glycol, 1,4-butanediol, trimethylolpropane, glycerin, polyoxyethylene glycol, polyoxypropylene glycol, and polyglycerin; polyglycidyl compounds such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether; haloepoxy compounds such as epichlorohydrin, epibromohydrin, and $\alpha$-methyl epi-chlorohydrin; isocyanate compounds such as 2,4-tolylene diisocyanate, and hexamethylene diisocyanate; oxetane compounds such as 3-methyl-3-oxetane methanol, 3-ethyl-3-oxetane methanol, 3-butyl-3-oxetane methanol, 3-methyl-3-oxetane ethanol, 3-ethyl-3-oxetane ethanol, and 3-butyl-3-oxetane ethanol; oxazoline compounds such as 1,2-ethylenebisoxazoline; carbonate compounds such as ethylene carbonate; hydroxyalkylamide compounds such as bis[N,N-di($\beta$-hydroxyethyl)]adipamide; and other crosslinking agents. These crosslinking agents may be used alone or two or more kinds thereof may be used in combination. The crosslinking agent may include a polyglycidyl compound, or may include at least one selected from the group consisting of (poly)ethylene glycol diglycidyl ether, (poly)glycerin diglycidyl ether, (poly)glycerin triglycidyl ether, (poly)propylene glycol polyglycidyl ether, and polyglycerol polyglycidyl ether.

The use amount of the surface crosslinking agent may be 0.00001 to 0.02 moles, 0.00005 to 0.01 moles, or 0.0001 to 0.005 moles with respect to 1 mole of the ethylenically unsaturated monomer used for the polymerization, from the viewpoint of exhibiting suitable water-absorbent characteristics by the obtained hydrogel-like polymer appropriately crosslinking.

After the surface crosslinking, the water and hydrocarbon dispersion medium may be removed by a known method to dry the surface-crosslinked hydrogel-like polymer. In this manner, polymer particles, a dried product of the surface-crosslinked hydrogel-like polymer, can be obtained.

The water-absorbent resin particle of the present embodiment may be formed of only the polymer particles, but can further contain various additional components selected from a gel stabilizing agent, a metal chelating agent, and a flowability improver (lubricant), for example. The additional components can be disposed inside the polymer particle or on the surface of the polymer particle, or both thereof. The additional components may be a flowability improver (lubricant). A flowability improver may be inorganic particles. Examples of the inorganic particles include silica particles such as amorphous silica.

The water-absorbent resin particle may contain a plurality of the inorganic particles disposed on the surface of the polymer particle. For example, the polymer particles and the inorganic particles can be mixed to dispose the inorganic particles on the surfaces of the polymer particles. These inorganic particles may be silica particles such as amorphous silica. In a case where the water-absorbent resin particle contains the inorganic particles disposed on the surfaces of the polymer particles, a proportion of the inorganic particles may be 0.01% by mass or more or 0.05% by mass or more, and 5.0% by mass or less, 1.0% by mass or less, or 0.5% by mass or less, with respect to the mass of the polymer particles. The inorganic particles herein usually have a minute size as compared with the size of the polymer particle. For example, the average particle diameter of the inorganic particles may be 0.1 to 50 $\mu$m, 0.5 to 30 $\mu$m, or 1 to 20 $\mu$m. The average particle diameter herein can be a value measured by a dynamic light scattering method or a laser diffraction/scattering method.

Since the water-absorbent resin particle according to the present embodiment has the water retention capacity + the water absorption amount under load 6.21 kPa of 56 g/g or more, the water-absorbent resin particle can absorb a large amount of liquid under a load. Furthermore, since the water-absorbent resin particle has a water retention capacity of 40 g/g or more and a water absorption rate of 50 seconds or less, the water-absorbent resin particle can instantly absorb a liquid (excess water) which cannot be absorbed by the water-absorbent sheet after liquid absorption. Therefore, the water-absorbent resin particle according to the present embodiment has the better effect of reducing gel leakage.

The gel leakage amount in a case of using the water-absorbent sheet formed of the water-absorbent resin particles according to the present embodiment may be 70 g or less or 68 g or less, and may be, for example, 60 to 70 g.

The gel leakage amount in a case where the water-absorbent resin particles are used for the water-absorbent sheet is measured by the following method. The water-absorbent resin particles in an amount of 3.0 g is uniformly scattered over the entire air-through-type porous liquid permeable sheet, which has a size of 12 cm × 8 cm and a basis weight of 22 g/m$^2$ and is made of polyethylene-polypropylene. The air-through-type porous liquid permeable sheet, which has a basis weight of 22 g/m$^2$ and is made of polyethylene-polypropylene, is disposed on a resin layer formed of the water-absorbent resin particles to cover the entire resin layer and disposed over the upper surface of a laminate to obtain a water-absorbent sheet for evaluation. The water-absorbent sheet for evaluation is placed on a horizontal plane in an environment of a temperature of 25±2°C and a humidity of 50±10%. A liquid injection cylinder is disposed at the center of the water-absorbent sheet for evaluation placed on the horizontal plane. The liquid injection cylinder is a cylinder, which is open at both ends and has a capacity of 100 mL with an inlet having an inner diameter of 3 cm. A liquid leakage prevention frame is disposed to surround the water-absorbent sheet for evaluation on which the liquid injection cylinder is disposed. The liquid leakage prevention frame has an opening portion of 12.5 cm × 8.5 cm and a height of 5 cm. 120 mL of artificial urine, the temperature of which is adjusted to 25 ± 1°C, is put into the liquid injection cylinder. The artificial urine is injected from the upper side of the center of the water-absorbent sheet for evaluation by 5 cm in the vertical direction by using a burette. The artificial urine is injected at a constant rate of 4 mL/sec. The liquid leakage prevention frame is removed from the liquid injection cylinder when the total amount of the artificial urine in the liquid injection cylinder is supplied from the liquid injection cylinder to the water-absorbent sheet for evaluation. Five minutes after the start of the artificial urine injection, a weight (12 cm × 8 cm) of 5.22 kg is placed on the main surface of the water-absorbent sheet for evaluation in contact with the liquid injection cylinder. The weight is removed one minute after the weight has been placed, and the air-through-type porous liquid permeable sheet on the side in contact with the weight is removed from the water-absorbent sheet. The weight of the gel pressed out from the air-through-type porous liquid permeable sheet side opposite to the side in contact with the weight is measured and defined as the gel leakage amount. The artificial urine is obtained by mixing and dissolving 5919.6 g of deionized water, 60.0 g of NaCl, 1.8 g of CaCl$_2$·H$_2$O, 3.6 g of MgCl$_2$·6H$_2$O, and 15.0 g of 1%-Triton X-100 to obtain a mixture and by mixing food blue No. 1 (for coloring) with the mixture.

The water-absorbent resin particle of the present embodiment has better absorbency for a body fluid such as urine and blood, and can be applied to the fields of sanitary products such as paper diapers, sanitary napkins, mild incontinence pads, and tampons, pet sheets, and materials for treating animal excrement such as dog or cat toilet litters, for example.

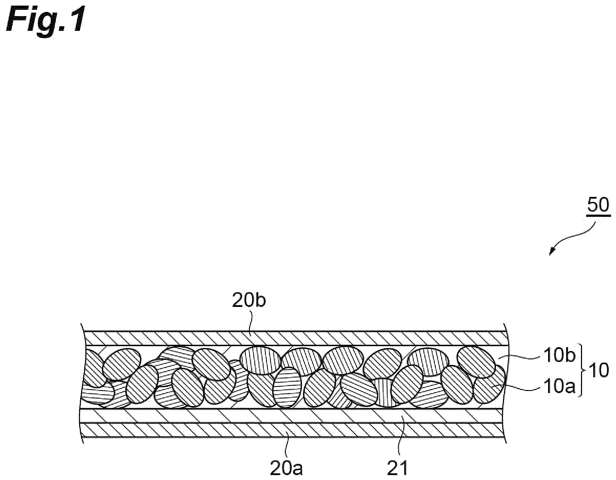

The above-mentioned water-absorbent resin particle is suitable for a water-absorbent sheet, for example. Fig. 1 is a cross-sectional view showing an example of a water-absorbent sheet. A water-absorbent sheet 50 shown in Fig. 1 has an absorber 10 and two core wrap sheets 20a and 20b. The core wrap sheets 20a and 20b are disposed at both sides of the absorber 10. In other words, the absorber 10 is disposed on the inner side of the core wrap sheets 20a and 20b. The shape of the absorber 10 is retained by being sandwiched between the two core wrap sheets 20a and 20b. The core wrap sheets 20a and 20b may be two sheets, may be one folded sheet, or may be one bag body.

The water-absorbent sheet 50 may further have an adhesive 21 interposed between the core wrap sheet 20a and the absorber 10. The adhesive 21 may be interposed not only between the core wrap sheet 20a and the absorber 10 but also between the core wrap sheet 20b and the absorber 10. The adhesive 21 is not particularly limited, and may be a hot melt adhesive, for example.

The absorber 10 has water-absorbent resin particles 10a according to the present embodiment mentioned above and a fiber layer 10b containing a fibrous material. The absorber 10 may not have the fiber layer 10b. A content of the water-absorbent resin particles in the absorber may be 70% by mass or more and 100% by mass or less, 80% by mass or more and 100% by mass or less, or 90% by mass or more and 100% by mass or less based on the mass of the absorber 10.

The thickness of the absorber 10 is not particularly limited, but in the dried state, the thickness may be 20 mm or less, 15 mm or less, 10 mm or less, 5 mm or less, 4 mm or less, 3 mm or less, or 2 mm or less, and may be 0.1 mm or more or 0.3 mm or more, for example. The mass of the absorber 10 per unit area may be 1000 $g/m^2$ or less, 800 $g/m^2$ or less, or 600 $g/m^2$ or less, or may be 100 $g/m^2$ or more.

The fibrous material constituting the fiber layer 10b can be, for example, cellulosic fibers, synthetic fibers, or a combination thereof. Examples of the cellulosic fibers include pulverized wood pulp, cotton, cotton linter, rayon, and cellulose acetate. Examples of the synthetic fibers include polyamide fibers, polyester fibers, and polyolefin fibers. The fibrous material may be hydrophilic fibers (for example, pulp).

The absorber 10 may not substantially include the fibrous material. A content of the fibrous material may be, for example, 10% by mass or less, 5% by mass or less, or 0% by mass based on the mass of the absorber 10. A content of the pulp in the absorber 10 may be within the above-mentioned range based on the mass of the absorber 10. Since the water-absorbent sheet 50 contains the water-absorbent resin particles according to the present embodiment, gel leakage is reduced even though the water-absorbent resin particles are contained in a high proportion.

The absorber 10 (or the fiber layer 10b) may further contain inorganic powder (for example, amorphous silica), a deodorant, an antibacterial agent, a fragrance, or the like. In a case where the water-absorbent resin particles 10a contain inorganic particles, the absorber 10 may contain inorganic powder in addition to the inorganic particles of the water-absorbent resin particles 10a.

The core wrap 20a is disposed on one surface side of the absorber 10 (upper side of the absorber 10 in Fig. 1) in a state of being in contact with the absorber 10. The core wrap 20b is disposed on the other surface side of the absorber 10 (on a lower side of the absorber 10 in Fig. 1) in a state of being in contact with the absorber 10. The absorber 10 is disposed between the core wrap 20a and the core wrap 20b. Examples of the core wraps 20a and 20b include tissues, non-woven fabrics, and the like. The core wrap 20a and the core wrap 20b each have, for example, a main surface having the same size as that of the absorber 10.

The water-absorbent sheet 50 is used for producing various absorbent articles, for example. Examples of the absorbent articles include diapers (for example, paper diapers), toilet training pants, incontinence pads, sanitary materials (sanitary napkins, tampons, and the like), sweat pads, pet sheets, portal toilet members, and animal excrement treatment materials.

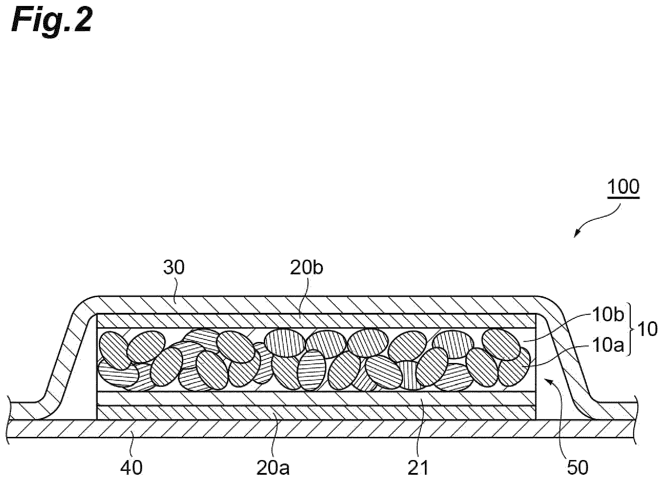

Fig. 2 is a cross-sectional view showing an example of an absorbent article. An absorbent article 100 shown in Fig. 2 includes a water-absorbent sheet 50, a liquid permeable sheet 30, and a liquid impermeable sheet 40. In the absorbent article 100, the liquid impermeable sheet 40, the water-absorbent sheet 50, and the liquid permeable sheet 30 are laminated in this order. In Fig. 2, there is a portion shown so that a gap is present between members, but the members may be in close contact with each other without the gap.

The liquid permeable sheet 30 is disposed on the outermost part at the side where the liquid to be absorbed enters. The liquid permeable sheet 30 is disposed on the core wrap 20a in a state of being in contact with the core wrap 20a. Examples of the liquid permeable sheet 30 include a non-woven fabric made of a synthetic resin such as polyethylene, polypropylene, polyester, and polyamide, and a porous sheet. The liquid impermeable sheet 40 is disposed on the outermost part at the opposite side to the liquid permeable sheet 30 in the absorbent article 100. The liquid impermeable sheet 40 is disposed on the lower side of the core wrap 20b in a state of being in contact with the core wrap 20b. Examples of the liquid impermeable sheet 40 include a sheet made of a synthetic resin such as polyethylene, polypropylene, and polyvinyl chloride, and a sheet made of a composite material of these synthetic resins and a non-woven fabric. The liquid permeable sheet 30 and the liquid impermeable sheet 40 each have, for example, a main surface wider than the main surface of the absorber 10, and outer edges of the liquid permeable sheet 30 and the liquid impermeable sheet 40 each extend around the absorber 10 and the core wraps 20a and 20b.

**Examples**

Hereinafter, the present invention will be more specifically described with reference to examples. However, the present invention is not limited to these examples.

[Production of Water-absorbent Resin Particles]

(Example 1)

A round-bottomed cylindrical separable flask having an inner diameter of 11 cm and an internal volume of 2 L equipped with a reflux cooling device, a dropping funnel, a nitrogen gas introduction tube, and a stirrer was prepared. As the stirrer, a stirrer blade having two stages of four inclined paddle blades with a blade diameter of 5 cm was used. 300 g of n-heptane as a hydrocarbon dispersion medium, 0.736 g of maleic acid-modified ethylene-propylene copolymer (Mitsui Chemicals, Inc., Hi-Wax 1105A) as a polymeric dispersant, and 0.736 g of sucrose stearic acid ester (HLB: 3, Mitsubishi-Chemical Foods Corporation, Ryoto Sugar Ester S-370) as a surfactant were added to the above-mentioned flask, and the mixture was stirred at a rotation speed of a stirrer of 550 rpm while being heated to 80°C, and the dispersant and surfactant were dissolved and then cooled to 60°C.

On the other hand, 92.0 g (1.03 mol) of a 80.5% by mass aqueous solution of acrylic acid serving as an ethylenically unsaturated monomer was charged in a beaker having an internal volume of 300 mL, 103.0 g of a 30% by mass aqueous solution of sodium hydroxide was added dropwise thereto while being cooled from the outside to perform 75% by mol of neutralization, and thereafter, 0.0736 g (0.272 mmol) of potassium persulfate serving as a peroxide, 0.0028 g (0.016 mmol) of ethylene glycol diglycidyl ether serving as an internal crosslinking agent, and 44.61 g of ion-exchanged water were added and dissolved to prepare a first stage monomer aqueous solution.

The prepared monomer aqueous solution was added to a separable flask, the inside of the flask was sufficiently replaced with nitrogen while stirring, and the flask was then immersed in a water bath at 70°C to be heated to allow the polymerization reaction to proceed for 30 minutes, thereby obtaining a first stage polymerization slurry solution.

On the other hand, 128.8 g (1.44 mol) of a 80.5% by mass aqueous solution of acrylic acid serving as an ethylenically unsaturated monomer was charged in another beaker having an internal volume of 500 mL, 144.2 g of a 30% by mass aqueous solution of sodium hydroxide was added dropwise thereto while being cooled from the outside to perform 75% by mol of neutralization, and thereafter, 0.1288 g (0.477 mmol) of potassium persulfate serving as a peroxide, 0.0090 g (0.052 mmol) of ethylene glycol diglycidyl ether serving as an internal crosslinking agent, and 16.64 g of ion-exchanged water were added and dissolved to prepare a second stage monomer aqueous solution.

While stirring is performed at a rotation speed of the stirrer of 1000 rpm, the above-mentioned separable flask system was cooled to 27°C, and then the total amount of the above-mentioned second stage monomer aqueous solution was added to the above-mentioned first stage polymerization slurry solution. Thereafter, the inside of the system was replaced with nitrogen for 30 minutes, the flask was again immersed in a water bath at 70°C to be heated to cause a polymerization reaction, the internal temperature reached 82°C, and 60 g of ion-exchanged water was then added thereto to obtain a hydrogel-like polymer. At this time, the moisture percentage calculated by the following expression was 169% by mass.

The moisture percentage (% by mass) of the hydrogel-like polymer is calculated by the following expression.

$$\text{Moisture percentage} = (\text{Ww/Ws}) \times 100$$

Ww: Water amount of the hydrogel-like polymer obtained by adding water amount added and water amount used if necessary when mixing a radical polymerization initiator, a flocculant, a surface crosslinking agent, or the like to an amount obtained by subtracting water amount discharged to the outside of the system in the step such as a drying step from water amount contained in a monomer aqueous solution before polymerization in entire polymerization step.
Ws: Solid content calculated from the charged amount of materials such as the ethylenically unsaturated monomer, the crosslinking agent, and the radical polymerization initiator, which constitute the hydrogel-like polymer.

Thereafter, the flask was immersed in an oil bath set at 125°C, and 305.3 g of water was extracted to the outside of the system while n-heptane is refluxed by azeotropic distillation of n-heptane and water.

Thereafter, 4.42 g (0.507 mmol) of a 2 mass% aqueous solution of ethylene glycol diglycidyl ether was added to the flask as a surface crosslinking agent, and the mixture was held at 82°C for 2 hours.

Thereafter, n-heptane was evaporated at 125°C and dried to obtain a dried product (polymer particles). This dried product was allowed to pass through a sieve having an opening of 850 µm, and 0.1% by mass of amorphous silica (Oriental Silicas Corporation, Tokusil NP-S) was mixed with the dried product to obtain 229.7 g of the water-absorbent resin particles. The median particle diameter of these particles was 336 µm.

(Example 2)

Water-absorbent resin particles in an amount of 230.8 g were obtained in the same manner as in Example 1, except that 311.3 g of water was extracted to the outside of the system by azeotropic distillation. The median particle diameter of the particles was 337 µm.

(Comparative Example 1)

Water-absorbent resin particles in an amount of 223.2 g were obtained in the same manner as in Example 1, except that the amount of the ethylene glycol diglycidyl ether used as an internal crosslinking agent for preparing the first stage monomer aqueous solution was changed to 0.0101 g (0.058 mmol), the amount of the potassium persulfate used as a peroxide for preparing the second stage monomer aqueous solution was changed to 0.1030 g (0.381 mmol), the amount of the ethylene glycol diglycidyl ether used as an internal crosslinking agent for preparing the second stage monomer aqueous solution was changed to 0.0116 g (0.067 mmol), the ion-exchanged water was not added after the polymerization of the second stage, and the moisture percentage of the obtained hydrogel-like polymer was 142% by mass (the moisture percentage of the hydrogel-like polymer when the heating is started was changed to 142% by mass), and 259.2 g of water was extracted to the outside of the system by azeotropic distillation. The median particle diameter of the water-absorbent resin particles was 380 µm.

(Comparative Example 2)

Water-absorbent resin particles in an amount of 224.1 g was obtained in the same manner as in Comparative Example 1, except that the addition amount of the potassium persulfate dissolved in the first stage monomer aqueous solution was 0.0184 g (0.068 mmol), the addition amount of the ethylene glycol diglycidyl ether was 0.0046 g (0.026 mmol), and 0.0920 g (0.339 mmol) of the azo-based compound, 2,2'-azobis(2-amidinopropane)dihydrochloride, were further added. In addition, the addition amount of the potassium persulfate dissolved in the second stage monomer aqueous solution was 0.0258 g (0.095 mmol), the addition amount of the ethylene glycol diglycidyl ether was 0.0116 g (0.067 mmol), and 0.1288 g (0.475 mmol) of the azo-based compound, 2,2'-azobis(2-amidinopropane)dihydrochloride, were further added. Furthermore, 232.3 g of water was extracted to the outside of the system by azeotropic distillation, and 0.2% by mass of amorphous silica was mixed with the dried product. The median particle diameter of the water-absorbent resin particles was 374 µm.

(Comparative Example 3)

Water-absorbent resin particles in an amount of 226.0 g were obtained in the same manner as in Comparative Example 1, except that 272.4 g of water was extracted to the outside of the system by azeotropic distillation. The median particle diameter of the water-absorbent resin particles was 362 µm.

[Water Retention Capacity]

The water retention capacity (room temperature, 25°C ± 2°C) of the water-absorbent resin particle in a physiological saline was measured by the following procedure. First, a cotton bag (cotton broadcloth No. 60, a width of 100 mm × a length of 200 mm) into which 2.0 g of the water-absorbent resin particles had been weighed was placed in a beaker having the internal volume of 500 mL. After 500 g of the physiological saline was poured into the cotton bag containing the water-absorbent resin particles at one time so that lumps could not be produced, the upper part of the cotton bag was bound with a rubber band, and the cotton bag was left to stand for 30 minutes to swell the water-absorbent resin particles. The cotton bag after an elapse of 30 minutes was dehydrated for 1 minute using a dehydrator (manufactured by KOKUSAN Co., Ltd., product number: H-122) which had been set to have the centrifugal force of 167 G, and a mass Wa [g] of the cotton bag containing the swollen gel after dehydration was then measured. The same operation was performed without addition of the water-absorbent resin particles, an empty mass Wb [g] at the time when the cotton bag was wet was measured, and the water retention capacity of the water-absorbent resin particle in the physiological saline was calculated from the following expression.

$$\text{Water retention capacity [g/g]} = (Wa - Wb)/2.0$$

[Water Absorption Amount under Load of 6.21 kPa]

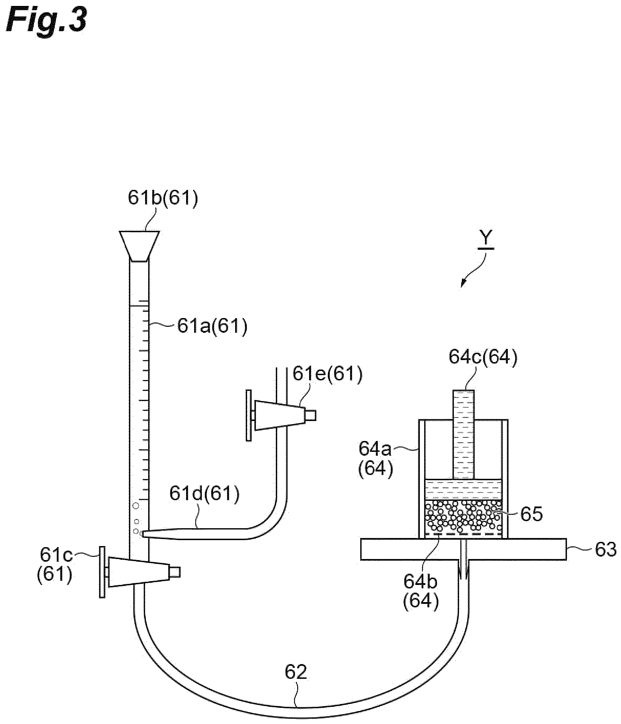

The water absorption amount of the water-absorbent resin particle in the physiological saline under a load (under pressurization) (room temperature, 25°C ± 2°C) was measured by using a measurement device Y shown in Fig. 3. The measurement device Y is formed of a burette unit 61, a conduit 62, a measurement table 63, and a measurement unit 64 placed on the measurement table 63. The burette unit 61 has a burette 61a extending in a vertical direction, a rubber stopper 61b disposed on the upper end of the burette 61a, a cock 61c disposed on the lower end of the burette 61a, an air introduction tube 61d having one end that extends into the burette 61a in the vicinity of the cock 61c, and a cock 61e disposed on the other end side of the air introduction tube 61d. The conduit 62 is attached between the burette unit 61 and the measurement table 63. The inner diameter of the conduit 62 is 6 mm. At the central portion of the measurement table 63, a hole having a diameter of 2 mm is formed, and the conduit 62 is connected thereto. The measurement unit 64 has a cylinder 64a (made of acrylic resin (plexiglass)), a nylon mesh 64b adhered to the bottom of the cylinder 64a, and a weight 64c. The inner diameter of the cylinder 64a is 20 mm. The opening of the nylon mesh 64b is 75 $\mu$m (200 mesh). At the time of measurement, water-absorbent resin particles 65 to be measured are uniformly scattered on the nylon mesh 64b. The weight 64c has a diameter of 19 mm and a mass of 179.4 g. The weight 64c having a mass of 179.4 g is placed on the water-absorbent resin particles 65, and a load of 6.21 kPa can be applied to the water-absorbent resin particles 65.

The water-absorbent resin particles 65 in an amount of 0.100 g was put into the cylinder 64a of the measurement device Y, followed by the weight 64c having a mass of 179.4 g being placed thereon to start measurement. The amount of reduction in the water level of the physiological saline inside the burette 61a corresponds to the amount of the physiological saline absorbed by the water-absorbent resin particles 65 because the same volume of air as that of the physiological saline absorbed by the water-absorbent resin particles 65 is quickly and smoothly supplied to the inside of the burette 61a from the air introduction tube. A scale of the burette 61a is engraved from top to bottom in increments of 0 mL to 0.5 mL; as the water level of the physiological saline, a scale Va of the burette 61a before the start of water absorption and a scale Vb of the burette 61a in 60 minutes from the start of the water absorption are read; and the water absorption amount under the load was calculated by the following expression.

Water absorption amount under load 6.21 kPa [g/g] = (Vb - Va) [mL] $\times$ 1.0028 [g/mL] (specific gravity of 0.9% by weight NaCl)/0.1 [g]

[Water Absorption Rate (Vortex Method)]

The water absorption rate of the water-absorbent resin particle with respect to the physiological saline was measured according to the following procedure based on the Vortex method. First, 50 ± 0.1 g of a physiological saline, the temperature of which was adjusted to a temperature of 25 ± 0.2°C in a constant temperature water tank, was measured in a beaker having a volume of 100 mL. Subsequently, a vortex was generated by stirring at a rotation speed of 600 rpm using a magnetic stirrer bar (8 mm$\varphi$ $\times$ 30 mm, without ring). 2.0 ± 0.002 g of the water-absorbent resin particles was added to the physiological saline at one time. The time period [seconds] from the time when the water-absorbent resin particles are added to the time when the vortex on the liquid surface is converged was measured, and the time period was recorded as the water absorption rate of the water-absorbent resin particle.

[Water Absorption Amount under Load of 2.07 kPa]

The same device as the measurement device Y used for measuring the water absorption amount under a load of 6.21 kPa was used, except that the mass of the weight 64c was 59.8 g. The weight 64c having a mass of 59.8 g is placed on the water-absorbent resin particles 65, and a load of 2.07 kPa can be applied to the water-absorbent resin particles 65.
The water-absorbent resin particles 65 in an amount of 0.100 g was put into the cylinder 64a of the measurement device Y, followed by the weight 64c having a mass of 59.8 g being placed thereon to start measurement. The amount of reduction in the water level of the physiological saline inside the burette 61a corresponds to the amount of the physiological saline absorbed by the water-absorbent resin particles 65 because the same volume of air as that of the physiological saline absorbed by the water-absorbent resin particles 65 is quickly and smoothly supplied to the inside of the burette 61a from the air introduction tube. A scale of the burette 61a is engraved from top to bottom in increments of 0 mL to 0.5 mL; as the water level of the physiological saline, a scale Va of the burette 61a before the start of water absorption and a scale Vb of the burette 61a in 60 minutes from the start of the water absorption are read; and the water absorption amount under the load was calculated by the following expression.

Water absorption amount under load 2.07 kPa [g/g] = (Vb - Va) [mL] × 1.0028 [g/mL] (specific gravity of 0.9% by weight NaCl)/0.1 [g]

(Median Particle Diameter)

The median particle diameter of the particles was measured according to the following procedure in an environment of room temperature (25 ± 2°C) and a humidity of 50 ± 10%. The continuous fully automatic sonic vibration-type sieving measuring apparatus (Robot shifter RPS-205, manufactured by Seishin Enterprise Co., Ltd.) was used to measure the particle size distribution of 5 g of the water-absorbent resin particles was measured with sieves having openings of 850 $\mu$m, 600 $\mu$m, 500 $\mu$m, 425 $\mu$m, 300 $\mu$m, 250 $\mu$m, and 180 $\mu$m and a tray in accordance with JIS standards. The relationship between the opening of the sieve and the integrated value of the mass percentage of the particles remaining on the sieve was plotted on logarithmic probability paper by integrating in the order from the one having the largest particle diameter on the sieve with respect to this particle size distribution. The plots on the probability paper are connected with a straight line to obtain the particle diameter corresponding to a cumulative mass percentage of 50% by mass as the median particle diameter.

[Performance of Absorber]

(Preparation of Artificial Urine)

A solution was prepared by blending and dissolving components in ion-exchanged water so that inorganic salts were present as shown below, and a small amount of Blue No. 1 was further blended therein to prepare artificial urine.

Composition of artificial urine

· Deionized water: 5919.6 g
· NaCl 60.0g
· $CaCl_2 \cdot H_2O$: 1.8 g
. $MgCl_2 \cdot 6H_2O$: 3.6 g
· Food Blue No. 1 (for coloration)
· 1%-TritonX-100 15.0g

(Production of Water-absorbent Sheet)

The water-absorbent resin particles in an amount of 3.0 g was uniformly scattered over the entire air-through-type porous liquid permeable sheet, which has a size of 12 cm × 8 cm and a basis weight of 22 g/m$^2$ and is made of polyethylene-polypropylene. Then, the air-through-type porous liquid permeable sheet, which has a basis weight of 22 g/m$^2$ and is made of polyethylene-polypropylene, was disposed on a resin layer formed of the water-absorbent resin particles to cover the entire resin layer and disposed over the upper surface of a laminate to obtain a water-absorbent sheet.

(Measurement of Gel Leakage Amount of Water-absorbent Sheet)

The water-absorbent sheet was placed on a horizontal plane of a working table in an environment of a temperature of 25±2°C and a humidity of 50±10%. Then, a liquid injection cylinder (cylinder with both ends open) having a volume of 100 mL and an inlet with an inner diameter of 3 cm was placed at the center of the water-absorbent sheet. Furthermore, a liquid leakage prevention frame having an opening portion of 12.5 cm × 8.5 cm and a height of 5 cm was placed to surround the water-absorbent sheet. Subsequently, 120 mL of artificial urine, the temperature of which is adjusted to 25±1°C, was injected (supplied from the vertical direction) into the cylinder from a height of 5 cm above the center by using a burette (4 mL/sec). After the artificial urine disappeared from the cylinder, the cylinder and the liquid leakage prevention frame were removed. The weight (12 cm × 8 cm) having a weight of 5.22 kg was placed 5 minutes after the start of injecting the artificial urine. After 1 minute, the weight was removed, and the non-woven fabric on the upper layer of the water-absorbent sheet was peeled off. The weight of the gel pressed out from the non-woven fabric on the lower layer of the water-absorbent sheet was measured and defined as the gel leakage amount.

[Table 1]

| Example Comparative Example | Performance of water-absorbent resin particle | | | | | | Performance of absorber |
| --- | --- | --- | --- | --- | --- | --- | --- |
| | Water retention capacity [g/g] | Water absorption amount under load [g/g] | | Water retention capacity + water absorption amount under load 2.07 kPa [g/g] | Water retention capacity + water absorption amount under load 6.21 kPa [g/g] | Water absorption rate [sec] | Gel leakage amount [g] |
| | | 2.07 kPa | 6.21 kPa | | | | |
| Example 1 | 44 | 38 | 14 | 82 | 58 | 37 | 67 |
| Example 2 | 49 | 35 | 12 | 84 | 61 | 40 | 67 |
| Comparative Example 1 | 42 | 34 | 13 | 76 | 55 | 42 | 77 |
| Comparative Example 2 | 49 | 37 | 15 | 86 | 64 | 58 | 77 |
| Comparative Example 3 | 50 | 15 | 4 | 65 | 54 | 44 | 71 |

The gel leakage of the water-absorbent sheet containing the water-absorbent resin particles of Examples was reduced.

**Reference Signs List**

10: Absorber

10a, 65: water-absorbent resin particle

10b: fiber layer

20a, 20b: core wrap

21: adhesive

30: liquid permeable sheet

40: liquid impermeable sheet

50: water-absorbent sheet

61: burette unit

61a: burette

61b: rubber stopper

61c, 61e: cock

61d: air introduction tube

62: conduit

63: measurement table

64: measurement unit

64a: cylinder

64b: nylon mesh

64c: weight

100: absorbent article

Y: measurement device

## Claims

1. A water-absorbent resin particle comprising:

   a crosslinked polymer having, as a monomer unit, at least one ethylenically unsaturated monomer selected from the group consisting of (meth)acrylic acid and a salt thereof,
   wherein a ratio of the (meth)acrylic acid and the salt thereof is 70% to 100% by mol with respect to a total amount of the monomer unit in the crosslinked polymer,
   a water retention capacity of the water-absorbent resin particle in a physiological saline is 40 g/g or more,
   a sum of a water retention capacity (g/g) of the water-absorbent resin particle in the physiological saline and a water absorption amount (g/g) of the water-absorbent resin particle under a load of 6.21 kPa is 56 g/g or more, and
   a water absorption rate of the water-absorbent resin particle is 50 seconds or less.

2. An absorber comprising the water-absorbent resin particle according to claim 1.

3. The absorber according to claim 2,
   wherein a content of the water-absorbent resin particle is 90% by mass or more and 100% by mass or less based on a mass of the absorber.

4. An absorbent article comprising the absorber according to claim 2 or 3.

# *Fig.1*

*Fig.2*

# Fig.3

61b(61)

Y

61a(61)

64c(64)

61e(61)

64a
(64)

61d(61)

65

61c
(61)

63

64b
(64)

62

## INTERNATIONAL SEARCH REPORT

International application No.

**PCT/JP2023/010409**

### A. CLASSIFICATION OF SUBJECT MATTER

**A61F 13/53**(2006.01)i; **A61F 13/531**(2006.01)i
FI:    A61F13/53 300; A61F13/531

According to International Patent Classification (IPC) or to both national classification and IPC

### B. FIELDS SEARCHED

Minimum documentation searched (classification system followed by classification symbols)

A61F13/53; A61F13/531

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | WO 2020/218168 A1 (SUMITOMO SEIKA CHEMICALS CO., LTD.) 29 October 2020 (2020-10-29) paragraphs [0020]-[0022], [0027], [0077] | 1-4 |

☐ Further documents are listed in the continuation of Box C.     ☑ See patent family annex.

| | |
|---|---|
| * Special categories of cited documents: <br> "A" document defining the general state of the art which is not considered to be of particular relevance <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
|---|---|
| **08 May 2023** | **23 May 2023** |

| Name and mailing address of the ISA/JP | Authorized officer |
|---|---|
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (January 2015)

International application No.

**PCT/JP2023/010409**

| Patent document cited in search report | Publication date (day/month/year) | Patent family member(s) | Publication date (day/month/year) |
|---|---|---|---|
| WO 2020/218168 A1 | 29 October 2020 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007514833 A